# NOUVEAU FASCICULE DE BREVET EUROPEEN

(11) **EP 1 404 288 B2**
(45) Date de publication et mention de la décision concernant l'opposition: **12.06.2019**
(45) Mention de la délivrance du brevet: 29.10.2008
(21) Numéro de dépôt: 01272053.8
(22) Date de dépôt: 26.11.2001
(51) Int. Cl.: A61Q 5/06, A61K 8/49, A61K 8/73

(54) **COMPOSITION POUR LA TEINTURE D'OXYDATION DES FIBRES KERATINIQUES COMPRENANT UN AMINO PYRAZOLE ET UN COMPOSE CELLULOSIQUE**
OXIDATIONSHAARFÄRBEMITTEL ENTHALTEND EIN AMINOPYRAZOL UND EINE CELLULOSISCHE VERBINDUNG
COMPOSITION FOR OXIDATION DYEING OF KERATINOUS FIBRES COMPRISING A AMINO PYRAZOLE AND A CELLULOSE COMPOUND

(30) Priorité: 22.12.2000 FR 0016953
(43) Date de publication de la demande: 07.04.2004
(73) Titulaire: L'Oréal, 75008 Paris (FR)
(72) Inventeur: COTTERET, Jean, F-78480 Verneuil-sur-Seine (FR)
(74) Mandataire: Fevrier, Murielle Françoise E.
(86) Numéro de dépôt international: PCT/FR2001/003728
(87) Numéro de publication internationale: WO 2002/051372

(56) Documents cités:
- EP-A1- 0 555 203
- EP-A2- 0 189 935
- WO-A1-00/61087
- WO-A1-01/97762
- WO-A1-98/17234
- WO-A1-98/20847
- WO-A1-99/17727
- DE-A- 4 133 957
- DE-A- 4 234 885
- DE-A1- 3 843 892
- DE-A1- 19 916 029
- DE-C- 19 730 412
- US-A- 4 243 802
- US-A- 5 061 289
- US-A- 5 430 159
- US-A- 5 534 267
- US-A- 5 735 908

## Description

La présente invention a pour objet une composition pour la teinture d'oxydation des fibres kératiniques, en particulier des fibres kératiniques humaines telles que les cheveux, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être choisis notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine.

Il a déjà été proposé, notamment dans les demandes de brevet allemand DE 3 843 892, DE 4 234 887, DE 4 234 886, DE 4 234 885 ou DE 195 43 988 des compositions pour la teinture d'oxydation des fibres kératiniques contenant à titre de base d'oxydation des dérivés de pyrazole tels que des 4,5-diamino pyrazoles, des 3,4-diamino pyrazoles ou des 3,4,5-triamino pyrazoles. De telles compositions ne sont cependant pas entièrement satisfaisantes car lors des processus de coloration se produisent des réactions secondaires qui peuvent avoir des effets négatifs du point de vue de l'innocuité et des propriétés des colorations obtenues et notamment de la puissance et de la ténacité des colorations vis à vis des diverses agressions que peuvent subir les cheveux.

L'invention a pour but de développer de nouvelles compositions tinctoriales ne présentant pas les inconvénients des teintures de la technique antérieure, en particulier, des teintures puissantes, particulièrement résistantes aux diverses agressions que peuvent subir les cheveux et présentant une bonne innocuité.

A cet effet, l'invention a pour objet une composition pour la teinture d'oxydation des fibres kératiniques humaines et en particulier des fibres kératiniques humaines telles que les cheveux, caractérisée par le fait qu'elle comprend, dans un milieu approprié pour la teinture:
- au moins une base d'oxydation choisie parmi le 4,5-diamino 1-hydroxyéthylpyrazoles et/ou un de ces sels d'addition avec un acide.
- au moins un composé cellulosique choisi parmi les hydroxyalkyl-afkylcelluloses choisies parmi les hydroxypropyl-méthylcelluloses, les hydroxyéthyl-méthylcelluloses, les hydroxyéthyl-éthylcelluloses et les hydroxybutyl-méthylcelluloses

La composition de teinture d'oxydation de l'invention permet d'obtenir avec une bonne innocuité des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes).

Sauf indication contraire, tous les radicaux, substituants, groupements chaines dans le cadre de l'invention sont linéaires ou ramifiés, substitués ou non.

Le ou les composés cellulosiques conformes à l'invention représentent de préférence de 0,00001 à 10 % en poids environ du poids total de la composition tinctoriale, encore plus préférentiellement de 0,001 à 5 % et encore plus préférentiellement de 0,001 à 3 % en poids environ de ce poids.

Le 4,5 diaminopyrazoles conformes à l'invention et/ou le ou les sels d'addition avec un acide correspondants représentent de préférence de 0,0005 à 12 % en poids environ du poids total de la composition tinctoriale, et plus préférentiellement de 0,005 à 6 % en poids environ de ce poids.

De préférence, le rapport pondéral du ou des composés cellulosiques avec le 4,5-diaminopyrazole et/ou le ou les sels d'addition avec un acide est compris entre 0,001 et 100 et encore plus préférentiellement entre 0,01 et 10.

Les compositions tinctoriales conformes à l'invention contiennent de préférence au moins un coupleur. Les coupleurs utilisables sont ceux classiquement utilisés pour la teinture d'oxydation et notamment les métaphénylènediamines, les métaaminophénols, les métadiphénols, les dérivés de naphtol et les coupleurs hétérocycliques.

Les méta-phénylènediamines, les méta-aminophénols et les méta-diphénols pouvant être utilisés à titre de coupleurs additionnels dans la composition tinctoriale conforme à l'invention sont de préférence choisis parmi les composés répondant à la formule (1) suivante et leurs sels d'addition avec un acide : 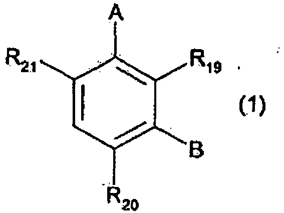 dans laquelle :
- A et B, identiques ou différents, représentent un radical hydroxyle, amino ou -NHR₂₂ dans lequel R₂₂ représente un radical alkyle en C₁-C₄, monohydroxyalkyle en C₁-C₄, ou polyhydroxyalkyle en C₂-C₄,
- R₁₉, R₂₀ et R₂₁, identiques ou différents, représentent un atome d'hydrogène ou d'halogène tel qu'un atome de brome, de chlore, d'iode ou de fluor, un radical alkyle en C₁-C₄, alcoxy en C₁-C₄, monohydroxyalkyle en C₁-C₄ ou polyhydroxyalkyle en C₂-C₄, monohydroxyalcoxy en C₁-C₄ ou polyhydroxyalcoxy en C₂-C₄.

Parmi les composés de formule (1) ci-dessus, on peut notamment citer le 2-méthyl 5-amino phénol, le 2-méthyl 5-amino 6-chloro phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 2,6bis(β-hydroxyéthylamino) toluène et leurs sels d'addition avec un acide.

Le ou les coupleurs hétérocycliques pouvant être utilisés à titre de coupleurs additionnels dans la composition tinctoriale conforme à l'invention peuvent notamment être choisis parmi les dérivés indoliques, les dérivés indoliniques, les dérivés pyridiniques, les dérivés pyrimidiniques, les pyrazolones, et leurs sels d'addition avec un acide. Parmi ces coupleurs hétérocycliques, on peut en particulier citer à titre d'exemple, le sésamol, le 1-N(β-hydroxyéthyl)amino 3'4-méthylènedioxy benzène,le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, la 3,5-diamino 2,6-diméthoxy pyridine, la 2-amino 3-hydroxy pyridine le 1-H 3-méthyl pyrazole 5-one, le 1-phényl 3-méthyl pyrazole 5-one, et leurs sels d'addition avec un acide. Parmi les dérivés de naphtol on peut citer l'α-naphtol, le 2-méthyl-1-naphtol.

Le ou les coupleurs additionnels représentent de préférence de 0,0001 à 10 % en poids environ du poids total de la composition tinctoriale et encore plus préférentiellement de 0,005 à 5 % en poids environ de ce poids.

Les compositions tinctoriales conformes à l'invention peuvent également contenir d'autres bases d'oxydation classiquement utilisées pour la teinture d'oxydation, différentes d'un diamino pyrazole et/ou des colorants directs notamment pour modifier les nuances ou les enrichir en reflets.

Les bases d'oxydation additionnelles utilisables dans le cadré de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques autres que les pyrazoles de l'invention ainsi que leurs sels d'addition avec un acide, et notamment :
- (I) les paraphénylènediamines de formule (2) suivante et leurs sels d'addition avec un acide : 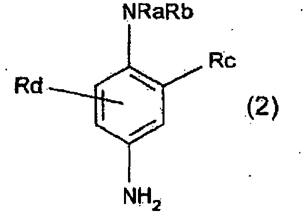 dans laquelle :
   **Rₐ** représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   **R_{b}** représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   **R_{c}** représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   **R_{d}** représente un atome d'hydrogène, d'halogène ou un radical alkyle en C₁-C₄.
   Rₐ et R_{b} peuvent également former avec l'atome d'hydrogène qui les porte un hétérocycle azoté à 5 ou 6 chaînons éventuellement substitué par un ou plusieurs groupements alkyle, hydroxy ou uréido.

   Parmi les groupements azotés de la formule (2) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (2) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-amino-N,N-bis-(β-hydroxyéthyl)-3-méthyl-aniline, la 4-amino-3-chloro-N,N-bis-(β-hydroxyéthyl)-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N, N-diméthyl-3-méthyl-paraphénylènediamine, la N,N-(éthyl, β-hydroxyéthyl)-paraphénylènediamine, la N-(β,γ-dihydroxypropyl)paraphénylènediamine, la N-(4'-aminophényl)-paraphénylènediamine, la N-phényl-paraphénylènediamine, la 2-β-hydroxyéthyloxy-paraphénylènediamine, et leurs sels d'addition avec un acide. Parmi les paraphénylènediamines de formule (2) ci-dessus, on préfère tout particulièrement la paraphénylènediamine, la paratoluylènediamine, la 2-isopropyl-paraphénylènediamine, la 2-β-hydroxyéthyl-paraphénylénediamine, la 2-(β-hydroxyéthyloxy-paraphénylènediamine, la 2,6-diméthyl-paraphénylènediamine, la 2,6-diéthyl-paraphénylènediamine, la 2,3-diméthyl-paraphénylènediamine, la N,N-bis-β-hydroxyéthyl)-paraphénylènediamine, la 2-chloro-paraphénylènediamine, et leurs sels d'addition avec un acide.
- (II) les bases doubles sont des composés comportant au moins deux noyaux aromatiques sur lesquels sont portés des groupements amino et/ou hydroxyle. Parmi les bases doubles utilisables à titre de bases d'oxydation dans les compositions tinctoriales conformes à l'invention, on peut notamment citer les composés répondant à la formule (3) suivante, et leurs sels d'addition avec un acide : 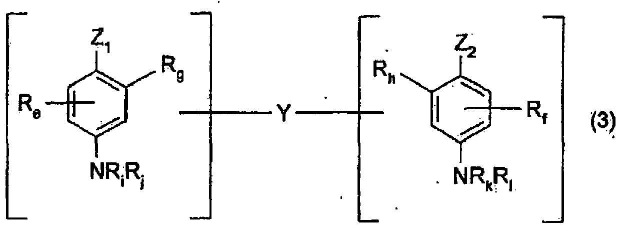 dans laquelle
   ∘ Z₁ et Z₂, identiques ou différents, représentent un radical hydroxyle ou -NH₂ pouvant être substitué par un radical alkyle en C₁-C₄ ou par un bras de liaison Y ;
   ∘ le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   ∘ Rₑ et R_{f} représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   ∘ R_{g}, Rₕ, Rᵢ, Rⱼ, Rₖ et Rₗ, identiques ou différents, représentent un atome d'hydrogène, un bras de liaison Y ou un radical alkyle en C₁-C₄ ;
   étant entendu que les composés de formule (3) ne comportent qu'un seul bras de liaison Y par molécule. Parmi les groupements azotés de la formule (3) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (3) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthyl-aminophényl)tétraméthylènediamine, la N,N'-bis-(éthyl) N,N'-bis-(4'-amino, 3'-méthylphényl)éthylènediamine, le 1,8-bis-(2,5-diaminophénoxy)-3,6-dioxaoctane, et leurs sels d'addition avec un acide. Parmi ces bases doubles de formule (3), le N,N'-bis-(β-hydroxyéthyl)N,N'-bis-(4'-aminophényl) 1,3-diamino propanol, le 1,8-bis-(2,5-diaminophénoxy)-3,6-dioxaoctane ou l'un de leurs sels d'addition avec un acide sont particulièrement préférés.
- (III) les para-aminophénols répondant à la formule (4) suivante, et leurs sels d'addition avec un acide : 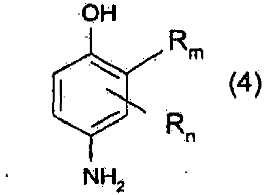 dans laquelle :
   **Rₘ** représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **Rₙ** représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (4) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1-,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol, le 2-(7-amino-pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl-pyrazolo-[1,5-a]-pyrimidine-3,7-diamine, la 2,6-diméthyl-pyrazolo-[1,5-a]-pyrimidine-3,7-diamine, la 2, 5, N7, N7-tetraméthyl-pyrazolo-[1,5-a]-pyrimidine-3,7-diamine, et leurs sels d'addition et leurs formes tautomères, lorsqu'il existe un équilibre tautomérique et leurs sels d'addition avec un acide.

Selon la présente invention, les bases d'oxydation additionnelles peuvent représenter de préférence de 0,0005 à 12% en poids environ du poids total de la composition tinctoriale D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (5) suivante : 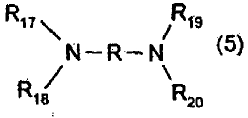 dans laquelle R est un reste propylène éventuellement substitué par un groupement hydroxyle ou un radical alkyle en C₁-C₄ ; R₁₇, R₁₈, R₁₉ et R₂₀, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant, cet agent oxydant pouvant être ajouté juste au moment de l'emploi à la composition tinctoriale ou au moyen d'une composition oxydante appliquée simultanément ou séquentiellement.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 60 minutes environ, de préférence 5 à 40 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates, percarbonates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition tinctoriale qui est appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Selon une variante, on applique sur ces fibres dans un premier temps une composition contenant au moins le composé cellulosique, et dans un deuxième temps une composition contenant au moins un diamino-pyrazole, l'application de la composition contenant le ou les composés cellulosiques étant ou non suivie par une étape de rinçage, la couleur étant révélée à l'aide d'un agent oxydant

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Selon un mode de réalisation différent, le dispositif comprend au moins trois compartiments, un premier compartiment qui contient le composé cellulosique utile pour l'invention, un deuxième compartiment qui contient un diaminopyrazole, et un troisième compartiment qui contient une composition oxydante.

Les exemples qui suivent sont destinés à illustrer l'invention sans pour autant en limiter la portée.

### EXEMPLES

### EXEMPLES DE TEINTURE 1 à 4

On a préparé les compositions tinctoriales, conformes à l'invention, suivantes (teneurs en grammes) :

| **EXEMPLES** | **1** | **2** | **3** | **4** |
|---|---|---|---|---|
| 4,5-diamino 1-β-hydroxyéthyl pyrazole, 2 HCl (base d'oxydation) | 0,645 | 0,645 | 0,645 | 0,645 |
| 3-amino 6-méthyl phénol (coupleur) | 0,369 | 0,369 | 0.369 | 0,369 |
| Cellulose en poudre vendue sous la dénomination CEPO S/100 par la Société SVENSKA (composé cellulosique selon l'invention) | 0,1 | - | - | - |
| Hydroxyéthylcellulose vendue sous la dénomination de NATROSOL 250 MR par la Société AQUALON (composé cellulosique selon l'invention) | - | 0,1 | - | - |
| Carboxyméthylcellulose, sel de sodium vendu sous la dénomination BLANOSE 7M8/SF par la Société AQUALON (composé cellulosique selon l'invention) | - | - | 0,15 | - |
| Hydroxyéthylcellulose quaternisée par le chlorure de glycidyl triméthylammonium vendue sous la dénomination NATROSOL ADX 237 par la Société HERCULES (composé cellulosique selon l'invention) | - | - | - | 0,2 |
| Support de teinture commun | (**) | (**) | (**) | (**) |
| Eau déminéralisée q.s.p. | 100 g | 100 g | 100 g | 100 g |
| (**) support de teinture commun contenant (M.A signifiant matière active) : | | | | |
| • Alcool oléique polyglycérolé à 2 moles de glycérol | | | 4,0 g | |
| • Alcool oléique polyglycérolé à 4 moles de glycérol, à 78 % de matières actives (M.A) | | | 5,69 g M.A. | |
| • Acide oléique | | | 3,0 g | |
| • Amine oléique à 2 moles d'oxyde d'éthylène vendue sous la dénomination commerciale ETHOMEEN 012 par la société AKZO | | | 7,0 g | |
| • Laurylamino succinamate de diéthylaminopropyle, sel de sodium, à 55 % de M.A. | | | 3,0 g M.A. | |
| • Alcool oléique | | | 5,0 g | |
| • Diéthanolamide d'acide oléique | | | 12,0 g | |
| • Propylèneglycol | | | 3,5 g | |
| • Alcool éthylique | | | 7,0 g | |
| • Dipropylèneglycol | | | 0,5 g | |
| • Monométhyléther de propylèneglycol | | | 9,0 g | |
| • Métabisulfite de sodium en solution aqueuse, à 35 % de M.A. | | | 0.455 g M.A. | |
| • Acétate d'ammonium | | | 0,8 g | |
| • Antioxydant, séquestrant | | | q.s. | |
| • Parfum, conservateur | | | q.s. | |
| • Ammoniaque à 20 % de NH₃ | | | 10 g | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

On obtient dans tous les exemples ci dessus une nuance rouge puissante et tenace avec une bonne innocuité.

## Revendications

1. Composition pour la teinture d'oxydation des fibres kératiniques humaines et en particulier des fibres kératiniques humaines telles que les cheveux, **caractérisée par le fait qu'**elle comprend, dans un milieu approprié pour la teinture :
- au moins une base d'oxydation choisie parmi le 4,5-diamino 1-hydroxyethyl pyrazole et un de ces sels d'addition avec un acide
• - au moins un composé cellulosique choisi parmi les hydroxyalkyl-alkylcelluloses choisies parmi les hydroxypropyl-méthylcelluloses, les hydroxyéthyl-méthylcelluloses, les hydroxyéthyl-éthylcelluloses et les hydroxybutyl-méthylcelluloses

2. Composition selon la revendication 1, **caractérisée en ce que** le ou les composés cellulosiques représentent de 0,00001 à 10 % en poids environ du poids total de la composition tinctoriale, de préférence de 0,001 à 5 %, et plus préférentiellement de 0,001 à 3 % en poids environ de ce poids.

3. Composition selon la revendication 1 ou 2, **caractérisée en ce que** le 4,5 diamino pyrazole et/ou les sels d'addition avec un acide correspondants représentent de 0,0005 à 12 % en poids du poids total de la composition tinctoriale.

4. Composition selon l'une quelconque des revendications précédentes 1 à 3, **caractérisée en ce que** le rapport pondéral du ou des composés cellulosiques avec le 4,5-diamino pyrazoles et/ou les sels d'addition avec un acide est compris entre 0,001 et 100 et de préférence entre 0,01 et 10.

5. Composition selon l'une quelconque des revendications précédentes 1 à 4, **caractérisée en ce que** les sels d'addition avec un acide sont choisis parmi les chlorhydrates, les bromhydrates, les sulfates et les tartrates, les lactates et les acétates.

6. Composition selon l'une quelconque des revendications précédentes 1 à 5, **caractérisée en ce qu'**elle contient au moins un coupleur.

7. Composition selon la revendication 6, **caractérisée en ce que** le ou les coupleurs représentent de 0,0001 à 10% en poids environ du poids total de la composition tinctoriale et encore plus préférentiellement de 0,005 à 5 % en poids environ de ce poids.

8. Composition selon l'une quelconque des revendications précédentes 1 à 7, **caractérisée par le fait qu'**elle contient au moins une base d'oxydation additionnelle autre que les pyrazoles définis dans la revendication 1.

9. Composition selon la revendication 8, **caractérisée en ce que** la ou les bases d'oxydation additionnelles représentent de 0,0005 à 12% en poids environ du poids total de la composition tinctoriale

10. Composition selon l'une quelconque des revendications précédentes 1 à 9, **caractérisée par le fait qu'**elle présente un pH compris entre 3 et 12.

11. Composition selon l'une quelconque des revendications précédentes 1 à 10, **caractérisée en ce qu'**elle se présente sous forme de liquides, de crèmes, de gels, ou sous toute autre forme appropriée pour réaliser une teinture des fibres kératiniques, et notamment des cheveux humains.

12. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 11, et que l'on révèle la couleur à pH acide, neutre ou alcalin à l'aide d'un agent oxydant.

13. Procédé selon la revendication 12, **caractérisé en ce que l'**agent oxydant présent dans la composition oxydante est choisi parmi le peroxyde d'hydrogène, le peroxyde d'urée, les bromates de métaux alcalins, les persels tels que les perborates, les percarbonates et persulfates, les peracides.

14. Procédé de teinture des fibres kératiniques et en particulier des fibres kératiniques humaines telles que les cheveux **caractérisé par le fait que** l'on applique sur ces fibres dans un premier temps une composition contenant au moins un composé cellulosique tel que défini dans l'une quelconque des revendications 1 à 20, dans un deuxième temps une composition contenant au moins un diamino-pyrazole tel que défini dans l'une quelconque des revendications 1 à 11, l'application de la composition contenant le ou les composés cellulosiques étant ou non suivie par une étape de rinçage, la couleur étant révélée à l'aide d'un agent oxydant.

15. Dispositif à plusieurs compartiments comprenant un premier compartiment renfermant une composition tinctoriale telle que définie à l'une quelconque des revendications 1 à 11 et un second compartiment renfermant une composition oxydante.

16. Dispositif à plusieurs compartiments comprenant un premier compartiment renfermant un composé cellulosique tel que défini dans l'une quelconque des revendications 1 à 11, un deuxième compartiment renfermant un diaminopyrazole tel que défini dans l'une quelconque des revendications 1 à 11, et un troisième compartiment renfermant une composition oxydante.

## Patentansprüche

1. Zusammensetzung zum oxidativen Färben von menschlichen Keratinfasern und insbesondere menschlichen Keratinfasern wie Haaren, **dadurch gekennzeichnet, dass** sie in einem zum Färben geeigneten Medium enthält:
- mindestens eine Oxidationsbase, die unter 4,5-Diamino-1-hydroxyethylpyrazol und einem seiner Additionssalze mit einer Säure ausgewählt ist,
- mindestens eine Celluloseverbindung, die unter Hydroxyalkylcellulosen ausgewählt ist, die aus Hydroxypropylmethylcellulosen, Hydroxyethylmethylcellulosen, Hydroxyethylethylcellulosen und Hydroxybutylmethylcellulosen ausgewählt sind.

2. Zusammensetzung nach Anspruch 1, **dadurch gekennzeichnet, dass** die Celluloseverbindung bzw. die Celluloseverbindungen etwa 0,00001 bis 10 Gew.-% des Gesamtgewichts der Färbezusammensetzung, vorzugsweise 0,001 bis 5 Gew.-% und noch weiter bevorzugt etwa 0,001 bis 3 Gew.-% dieses Gewichts ausmachen.

3. Zusammensetzung nach Anspruch 1 oder 2, **dadurch gekennzeichnet, dass** das 4,5-Diaminopyrazol und/oder die entsprechenden Additionssalze mit einer Säure 0,0005 bis 12 Gew.-% des Gesamtgewichts der Färbezusammensetzung ausmachen.

4. Zusammensetzung nach einem der vorhergehenden Ansprüche 1 bis 3, **dadurch gekennzeichnet, dass** das Gewichtsverhältnis der Celluloseverbindung bzw. der Celluloseverbindungen und des 4,5-Diaminopyrazols und/oder der Additionssalze mit einer Säure zwischen 0,001 und 100 und vorzugsweise zwischen 0,01 und 10 liegt.

5. Zusammensetzung nach einem der vorhergehenden Ansprüche 1 bis 4, **dadurch gekennzeichnet, dass** die Additionssalze mit einer Säure unter den Hydrochloriden, Hydrobromiden, Sulfaten, Tartraten, Lactaten und Acetaten ausgewählt sind.

6. Zusammensetzung nach einem der vorhergehenden Ansprüche 1 bis 5, **dadurch gekennzeichnet, dass** sie mindestens einen Kuppler enthält.

7. Zusammensetzung nach Anspruch 6, **dadurch gekennzeichnet, dass** der Kuppler bzw. die Kuppler etwa 0,0001 bis 10 Gew.-% des Gesamtgewichts der Färbezusammensetzung und noch weiter bevorzugt etwa 0,005 bis 5 Gew.-% dieses Gewichts ausmachen.

8. Zusammensetzung nach einem der vorhergehenden Ansprüche 1 bis 7, **dadurch gekennzeichnet, dass** sie mindestens eine zusätzliche Oxidationsbase enthält, die von den in Anspruch 1 definierten Pyrazolen verschieden ist.

9. Zusammensetzung nach Anspruch 8, **dadurch gekennzeichnet, dass** die zusätzliche Oxidationsbase bzw. die zusätzlichen Oxidationsbasen etwa 0,0005 bis 12 Gew.-% des Gesamtgewichts der Färbezusammensetzung ausmachen.

10. Zusammensetzung nach einem der vorhergehenden Ansprüche 1 bis 9, **dadurch gekennzeichnet, dass** sie einen pH-Wert zwischen 3 und 12 aufweist.

11. Zusammensetzung nach einem der vorhergehenden Ansprüche 1 bis 10, **dadurch gekennzeichnet, dass** sie in Form von Flüssigkeiten, Cremes, Gelen oder beliebigen anderen Formen, die zur Färbung von Keratinfasern und insbesondere menschlichen Haaren geeignet sind, vorliegt.

12. Verfahren zum Färben von Keratinfasern und insbesondere menschlichen Keratinfasern wie Haaren, **dadurch gekennzeichnet, dass** man auf diese Fasern mindestens eine Färbezusammensetzung gemäß einem der Ansprüche 1 bis 11 aufbringt und die Farbe bei saurem, neutralem oder alkalischem pH-Wert mit Hilfe eines Oxidationsmittels entwickelt.

13. Verfahren nach Anspruch 12, **dadurch gekennzeichnet, dass** das in der oxidierenden Zusammensetzung vorliegende Oxidationsmittel unter Wasserstoffperoxid, Harnstoffperoxid, Alkalimetallbromaten, Persalzen wie Perboraten, Percarbonaten und Persulfaten und Persäuren ausgewählt wird.

14. Verfahren zum Färben von Keratinfasern und insbesondere menschlichen Keratinfasern wie Haaren, **dadurch gekennzeichnet, dass** man auf die Fasern in einem ersten Schritt eine Zusammensetzung, die mindestens eine Celluloseverbindung gemäß einem der Ansprüche 1 bis 20 enthält, aufbringt und in einem zweiten Schritt eine Zusammensetzung, die mindestens ein Diaminopyrazol gemäß einem der Ansprüche 1 bis 11 enthält, aufbringt, wobei nach dem Aufbringen der die Celluloseverbindung bzw. die Celluloseverbindungen enthaltenden Zusammensetzung gegebenenfalls ein Spülschritt folgt, wobei die Farbe mit Hilfe eines Oxidationsmittels entwickelt wird.

15. Vorrichtung mit mehreren Kompartimenten, die ein erstes Kompartiment, das eine Färbezusammensetzung gemäß einem der Ansprüche 1 bis 11 enthält, und ein zweites Kompartiment, das eine oxidierende Zusammensetzung enthält, aufweist.

16. Vorrichtung mit mehreren Kompartimenten, die ein erstes Kompartiment, das eine Celluloseverbindung gemäß einem der Ansprüche 1 bis 11 enthält, ein zweites Kompartiment, das ein Diaminopyrazol gemäß einem der Ansprüche 1 bis 11 enthält, und ein drittes Kompartiment, das eine oxidierende Zusammensetzung enthält, aufweist.

## Claims

1. Composition for the oxidation dyeing of human keratin fibres, and in particular of human keratin fibres such as the hair, **characterized in that** it comprises, in a medium suitable for dyeing:
- at least one oxidation base chosen from 4,5-diamino-1-hydroxyethylpyrazole or an addition salt thereof with an acid;
- at least one cellulosic compound chosen from hydroxyalkylcelluloses chosen from hydroxypropylmethylcelluloses, hydroxyethylmethylcelluloses, hydroxyethylethylcelluloses and hydroxybutylmethylcelluloses.

2. Composition according to Claim 1, **characterized in that** the cellulosic compound(s) represent (s) from 0.00001% to 10% by weight approximately of the total weight of the dye composition, preferably from 0.001% to 5%, and more preferably from 0.001% to 3% by weight approximately of this weight.

3. Composition according to Claim 1 or 2, **characterized in that** the 4,5-diaminopyrazole and/or the corresponding addition salts with an acid represent from 0.0005% to 12% by weight of the total weight of the dye composition.

4. Composition according to any one of the preceding Claims 1 to 3, **characterized in that** the ratio by weight of the cellulosic compound(s) to the 4,5-diaminopyrazole and/or the addition salts with an acid is between 0.001 and 100, and preferably between 0.01 and 10.

5. Composition according to any one of the preceding Claims 1 to 4, **characterized in that** the addition salts with an acid are chosen from the hydrochlorides, the hydrobromides, the sulfates and the tartrates, the lactates and the acetates.

6. Composition according to any one of the preceding Claims 1 to 5, **characterized in that** it contains at least one coupler.

7. Composition according to Claim 6, **characterized in that** the coupler(s) represent(s) from 0.0001% to 10% by weight approximately of the total weight of the dye composition, and even more preferably from 0.005% to 5% by weight approximately of this weight.

8. Composition according to any one of the preceding Claims 1 to 7, **characterized in that** it contains at least one additional oxidation base other than the pyrazoles defined in Claim 1.

9. Composition according to Claim 8, **characterized in that** the additional oxidation base(s) represent(s) from 0.0005% to 12% by weight approximately of the total weight of the dye composition.

10. Composition according to any one of the preceding Claims 1 to 9, **characterized in that** it has a pH of between 3 and 12.

11. Composition according to any one of the preceding Claims 1 to 10, **characterized in that** it is in the form of liquids, creams or gels, or in any other form suitable for dyeing keratin fibres, and in particular human hair.

12. Method for dyeing keratin fibres, and in particular human keratin fibres such as the hair, **characterized in that** at least one dye composition as defined in any one of Claims 1 to 11 is applied to these fibres, and **in that** the colour is revealed at acidic, neutral or alkaline pH using an oxidizing agent.

13. Method according to Claim 12, **characterized in that** the oxidizing agent present in the oxidizing composition is chosen from hydrogen peroxide, urea peroxide, alkali metal bromates, persalts such as perborates, percarbonates and persulfates, and peracids.

14. Method for dyeing keratin fibres, and in particular human keratin fibres such as the hair, **characterized in that** the following are applied to these fibres: in a first step, a composition containing at least one cellulosic compound as defined in any one of Claims 1 to 20, and in a second step, a composition containing at least one diaminopyrazole as defined in any one of Claims 1 to 11, the application of the composition containing the cellulosic compound(s) possibly being followed by a rinsing step, the colour being revealed using an oxidizing agent.

15. Multicompartment device comprising a first compartment containing a dye composition as defined in any one of Claims 1 to 11, and a second compartment containing an oxidizing composition.

16. Multicompartment device comprising a first compartment containing a cellulosic compound as defined in any one of Claims 1 to 11, a second compartment containing a diaminopyrazole as defined in any one of Claims 1 to 11, and a third compartment containing an oxidizing composition.
